# FASCICULE DE BREVET EUROPEEN

(11) **EP 0 761 237 B1**
(45) Date de publication et mention de la délivrance du brevet: **05.04.2000**
(21) Numéro de dépôt: 95810558.7
(22) Date de dépôt: 08.09.1995
(51) Int. Cl.: A61L 2/20, A61L 11/00

(54) **Dispositif et procédé de traitement de matériaux, notamment en vue de leur décontamination**
Vorrichtung und Verfahren zur Desinfektion von Materialien
Device and method for disinfecting materials

(43) Date de publication de la demande: 12.03.1997
(73) Titulaire: BOX O3 International, 3073 Gümligen (CH)
(72) Inventeur: Duroselle Patrick, F-44800 Saint-Herblain (FR); Laberge, Fabrice, CH-1700 Fribourg (CH)
(74) Mandataire: BOVARD AG - Patentanwälte

(56) Documents cités:
- WO-A-86/05100
- DE-C- 3 813 793
- FR-A- 2 695 828
- US-A- 4 156 652
- US-A- 4 309 388

## Description

La présente invention concerne un dispositif de traitement de matériaux selon le préambule de la revendication 1, et un procédé de traitement de matériaux selon le préambule de la revendication 8. Plus spécifiquement, cette invention concerne le problème de la décontamination, de la désinfection et de la stérilisation de matériaux, par exemple de déchets provenant du milieu médical ou de matériaux utilisés dans l'industrie chimique, pharmaceutique ou agro-alimentaire.

Les praticiens des professions médicales et paramédicales produisent toute sorte de détritus de nature physique diverse, tels que plastiques, textiles, métaux, verre, liquides, etc.., susceptibles d'être contaminés par des germes microbiens ou viraux. Ces déchets peuvent par exemple être des aiguilles, des tubes en plastique, des compresses, des poches de sang, des tissus organiques divers, etc..., ne pouvant pas être éliminés comme des déchets ordinaires en raison des risques de contamination. D'autres professions génèrent également constamment des quantités croissantes de matériaux qu'il est nécessaire de décontaminer, de stériliser ou de désinfecter. C'est par exemple le cas de l'industrie chimique ou agro-alimentaire, ou des installations de traitement des déchets ou des eaux.

La quantité et la variété des déchets produits est en constante augmentation, notamment dans le domaine médical. Différentes méthodes et dispositifs de traitement de ce type de déchets ont par conséquent été développés pour faire face à cette demande. On connaît par exemple des dispositifs d'incinération de déchets qui font appel à la chaleur pour éliminer les germes microbiens. Ces dispositifs sont cependant mal adaptés à un traitement local des déchets, par exemple à un traitement dans les locaux du praticien. D'autres solutions font appel à une immersion dans une solution désinfectante, ou à un traitement par solution ionisante ou par ultrasons. Tous ces dispositifs, bien que fonctionnant de manière généralement satisfaisante, sont onéreux et encombrants et par conséquent réservés principalement aux collectivités.

On connaît également des dispositifs de décontamination au moyen de l'ozone ou d'un gaz enrichi en ozone. Ces dispositifs sont en particulier bien adaptés à la décontamination ou à la stérilisation de déchets médicaux à proximité du lieu de production.

La demande de brevet WO86/05100 décrit un dispositif de stérilisation de matériel médical au moyen d'ozone gazeux. Les déchets sont placés dans une enceinte de traitement mise sous vide. Un générateur d'ozone injecte de l'ozone dans l'enceinte, et des moyens de destruction sont prévus pour l'ozone résiduel. Le générateur d'ozone fonctionne à partir d'une source d'oxygène pur, ce qui entraîne un certain nombre de risques au niveau de l'installation, notamment de risques d'explosion si des déchets contenant de la graisse sont mis en contact direct avec l'oxygène. En outre, le générateur d'ozone fonctionne à partir d'un flux gazeux à fort taux d'humidité, ce qui nuit à son rendement.

Le dispositif décrit dans la demande FR-A-2'611'343 présente des inconvénients similaires, l'ozone étant toujours produit à partir d'oxygène pur et injecté directement dans l'enceinte de traitement.

Le dispositif de la demande de brevet FR-A-1'137'268 représente une amélioration sur le plan de la sécurité, car l'ozone est produit à partir d'air plutôt que d'oxygène pur. Cependant, ce dispositif ne permet pas d'obtenir les concentrations élevées en ozone qui sont nécessaires pour une décontamination ou une stérilisation poussée des déchets.

D'autres dispositifs de décontamination qui utilisent de l'ozone ou de l'air ozoné sont décrits par exemple dans les brevets ou demandes de brevets US-4'156'652, US-5'087'419 ou EP-A-2'81'870. Ces dispositifs utilisent généralement un circuit fermé établi entre un générateur d'ozone et une enceinte de traitement dans laquelle sont disposés les déchets à traiter. Certains prévoient en outre des moyens d'injection d'eau ou d'agitation du milieu gazeux ou des déchets, toujours pour faciliter l'action de l'ozone et d'améliorer sa pénétration. L'ensemble de ces dispositions entraîne un fonctionnement des générateurs dans de mauvaises conditions puisqu'ils sont parcourus par un flux gazeux humide et/ou pollué par l'enceinte de traitement et son contenu.

Enfin, la demande de brevet WO94/06483 (ou FR-A-2 695 828) décrit un dispositif de décontamination utilisant une enceinte d'accumulation d'air ozoné, mise en circuit fermé avec le générateur d'ozone. Ce circuit permet d'obtenir des concentrations élevées d'ozone dans l'air à l'intérieur de l'enceinte d'accumulation. En outre, les générateurs d'ozone peuvent travailler dans de bonnes conditions, puisqu'ils sont parcourus par un air propre et sec. Cet air à fort concentration d'ozone est régulièrement soutiré par une pompe de transfert, et injecté dans une enceinte de traitement indépendante de l'enceinte d'accumulation, et dans laquelle sont placés les déchets à décontaminer. La pompe de transfert prévue entre l'enceinte d'accumulation et l'enceinte de traitement doit par conséquent travailler dans un air très riche en ozone, ce qui ne va pas sans poser de nombreux problèmes. La pompe doit donc avoir une construction coûteuse pour résister à l'agressivité de ce gaz. En outre, la pression d'air ozoné, constante dans l'ensemble du dispositif et durant tout le processus, ne permet pas d'obtenir une pénétration en profondeur de l'air ozoné dans la masse de déchets à traiter. De surcroît, le dispositif continue de fonctionner même si l'enceinte de traitement présente un défaut d'étanchéité, ce qui pose un problème de sécurité.

L'expérimentation montre que la destruction spontanée de l'ozone, soit catalytique soit par combinaison avec les déchets à traiter, est extrêmement rapide (à peine quelques secondes). La quantité d'ozone disponible dans l'enceinte de traitement diminue donc rapidement. Pour compenser cette diminution, il est nécessaire d'injecter en permanence des quantités importante d'ozone dans l'enceinte de traitement.

### Buts de l'invention

Un but de la présente invention est donc de proposer un dispositif et un procédé de traitement qui ne présente pas les inconvénients mentionnés des dispositifs de l'art antérieur.

En particulier, un but est de proposer un dispositif de traitement dans lequel les moyens de génération d'ozone peuvent travailler avec de l'air propre et sec, et qui ne nécessite pas de pompe fonctionnant avec de l'air riche en ozone.

Un autre but est d'améliorer l'efficacité et/ou la rapidité du traitement, en améliorant la pénétration de l'air ozoné dans les déchets.

Un autre but est d'améliorer l'efficacité et/ou la rapidité du traitement, en utilisant des concentrations élevées d'ozone.

Un autre but est d'améliorer la sécurité offerte par le dispositif.

Ces buts sont atteints notamment grâce aux éléments de la partie caractérisante des revendications 1 et 8, des modes de réalisation préférentiels étant notamment décrits dans les revendications dépendantes.

En particulier, ces buts sont atteints grâce a des moyens pour créer une dépression dans l'enceinte de traitement. Cette dépression permet d'une part d'améliorer la pénétration de l'air ozoné dans les déchets à traiter, et d'autre part d'obtenir une injection brutale de l'ozone dans l'enceinte de traitement, sans nécessiter de pompe d'injection entre l'enceinte d'accumulation et l'enceinte de traitement.

Selon une autre caractéristique de l'invention, ces buts sont atteints par l'utilisation non pas d'une pompe de transfert travaillant dans l'air ozoné entre les enceintes d'accumulation et de traitement mais d'une pompe à vide située en aval du dispositif de destruction d'ozone, et créant une dépression dans l'enceinte de traitement. Cette pompe ne travaille ainsi que dans l'air.

Selon une autre caractéristique de l'invention, les moyens de génération d'ozone sont placés en circuit ouvert avec l'enceinte d'accumulation d'air ozoné. Cette disposition permet d'obtenir une augmentation de la pression dans l'enceinte d'accumulation au fur et à mesure de l'augmentation de concentration en ozone.

Selon l'invention, le dispositif comporte une vanne d'injection interposée entre l'enceinte d'accumulation d'air ozoné et l'enceinte de traitement, ladite vanne d'injection étant asservie de manière à déclencher l'injection brutale d'air ozoné dans l'enceinte de traitement dès que la pression à l'intérieur de l'enceinte de traitement est en dessous d'un seuil déterminé. Cette vanne permet ainsi de créer une alternance de cycles d'injection brutale d'air ozoné et de dépression dans l'enceinte de traitement, ce qui contribue à améliorer l'efficacité du dispositif pour une production horaire donnée des générateurs d'ozone.

Selon l'invention, le dispositif comporte aussi une vanne située en amont de l'enceinte d'accumulation pour mettre cette enceinte en communication avec l'atmosphère.

L'air ozoné peut être injecté brutalement dans l'enceinte de traitement, en raison du gradient de pression entre l'enceinte d'accumulation et l'enceinte de traitement au moment de l'ouverture de la vanne d'injection. Ce gradient de pression permet un effet d'injection brutale et tourbillonnaire d'air ozoné, améliorant la pénétration du mélange gazeux.

L'alternance de phases brèves d'injection d'air à forte concentration en ozone, puis de dépression dans l'enceinte de traitement, est plus efficace que l'injection continue, en raison de la destruction spontanée de l'ozone. De cette manière en effet, la concentration d'ozone dans l'enceinte de traitement reste supérieure au seuil d'activité biologique durant toute l'opération de traitement.

L'injection d'ozone n'est autorisée que si la dépression dans l'enceinte de traitement atteint une valeur déterminée dans un temps déterminé, ce qui représente un facteur de sécurité capital, l'injection ne pouvant se produire que si le dispositif de traitement est parfaitement étanche.

De plus, le fait de travailler dans l'enceinte de traitement en dépression ou à pression atmosphérique minimise les risques de fuites importantes.

D'autres caractéristiques et avantages de l'invention apparaîtront encore à la lecture de la description qui suit donnée à titre d'exemple préférentiel et illustrée par les figures qui montrent:
- La figure 1 un schéma d'ensemble du dispositif de traitement,
- La figure 2 une vue de détail de l'enceinte d'accumulation,
- La figure 3 une variante du circuit de purge monté en circuit fermé.

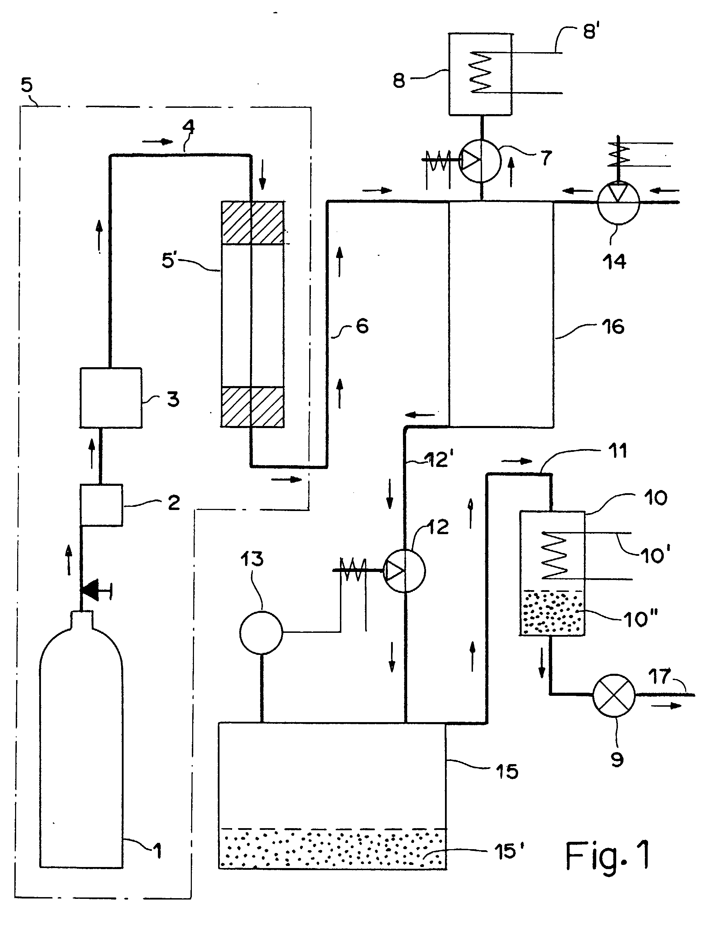

Dans le mode de réalisation de l'invention illustré par la figure 1, le dispositif de décontamination comprend essentiellement des moyens de génération d'ozone 5, une enceinte d'accumulation d'air ozoné 16, une enceinte de traitement 15 des déchets à traiter 15', ainsi que des moyens principaux de destruction d'ozone 10. Ces différents éléments sont reliés les uns aux autres par un ensemble de tubulures et de vannes permettant de mettre en oeuvre le procédé de décontamination.

Les moyens de génération d'ozone 5 comportent de manière préférentielle une source d'air comprimé 1, un détendeur débimétrique 2, un dispositif de séchage 3, une tubulure 4 et un générateur d'ozone 5'. Tous ces éléments sont connus; dans une variante, on pourrait utiliser de l'air ambiant plutôt qu'une source d'air comprimé et un détendeur. L'air propre provenant de la source 1 parvient dans le détendeur débimétrique 2, est desséché dans le dispositif de séchage 3 et est conduit par la tubulure 4 dans le générateur d'ozone 5'. Celui-ci est de type connu, par exemple du type fil-cylindre décrit par exemple dans la demande de brevet mentionnée EP-A-664715. Comme il travaille avec de l'air propre et sec, son efficacité est très élevée.

L'air enrichi en ozone sort du générateur d'ozone 5' par la canalisation 6 et pénètre dans la partie supérieure de l'enceinte d'accumulation 16. Les moyens de génération d'air ozoné 5 sont placés en boucle ouverte avec l'enceinte d'accumulation. Dans cette enceinte, la pression augmente donc en même temps que la concentration en ozone.

Au-delà d'une pression prédéterminée, une soupape de sûreté 7 s'ouvre afin d'évacuer l'excès d'air ozoné vers un destructeur d'ozone auxiliaire 8, de type connu, comportant une résistance électrique de chauffage 8'. Les surpressions accidentelles ou momentanées dans l'enceinte d'accumulation n'entraînent par conséquent pas de libération d'ozone dans l'atmosphère.

Une tubulure 12', fermée par une électrovanne 12, relie la partie inférieure de l'enceinte d'accumulation 16 avec l'enceinte de traitement 15 étanche, en acier inoxydable ou en tout autre matériau approprié, dans laquelle sont placés les produits à traiter 15'. L'enceinte est fermée par une porte ou un couvercle étanche permettant d'y introduire les déchets. La pompe à vide 9 établit une dépression dans le destructeur d'ozone principal 10. Celui-ci, de type connu, comporte de préférence une résistance de chauffage électrique 10' et un destructeur catalytique 10'' formant filtre antibactérien. L'air débarrassé par le destructeur 10 de l'ozone résiduel est évacué dans l'air libre par la tubulure de purge 17, en circuit ouvert.

La dépression dans le destructeur d'ozone principal 10 se propage par la tubulure 11 à l'enceinte de traitement 15 et aux matériaux à traiter 15'. A une valeur de dépression choisie, mesurée par un vacustat 13, l'électrovanne 12 qui lui est asservie s'ouvre brusquement. Dans une variante, l'électrovanne 12 s'ouvre dès que la différence de pression entre l'enceinte d'accumulation et l'enceinte de traitement dépasse un seuil déterminé. L'air ozoné en surpression contenu dans l'enceinte d'accumulation 16 pénètre alors brutalement dans l'enceinte de traitement 15. L'injection est de type tourbillonnaire, améliorant ainsi la pénétration de l'air ozoné dans les déchets 15'. Une temporisation, non représentée, ouvre l'électrovanne 14 une ou deux secondes après l'ouverture de l'électrovalve 12, mettant alors momentanément à l'air libre l'enceinte d'accumulation d'air ozoné 16.

Le piston pneumatique provoqué par l'ouverture de l'électrovalve 14, située dans la partie haute de l'enceinte d'accumulation d'air ozoné 16, chasse la plus grande partie de l'ozone contenu dans l'enceinte d'accumulation 16 qui, par densité, a tendance à s'accumuler dans sa partie inférieure. Cette ouverture rétablit la pression atmosphérique dans l'enceinte d'accumulation 16, la tubulure 12', l'enceinte de traitement 15, la tubulure 11 et le destructeur d'ozone principal 10.

Une seconde temporisation, non représentée, referme les électrovannes 14 et 12 après quelques secondes, démarrant un nouveau cycle d'accumulation d'air ozoné dans l'enceinte d'accumulation 16. Des moyens non représentés peuvent être prévus dans l'enceinte de traitement 15 pour agiter et/ou humidifier avant le prochain cycle de dépression et d'injection les matériaux à traiter 15' et/ou le milieu gazeux dans l'enceinte de traitement, afin d'améliorer l'efficacité de l'ozone.

Les déchets 15' sont de préférence broyés avant d'être introduits dans l'enceinte de traitement 15. Dans une variante, cette enceinte peut comporter un dispositif de broyage intégré. L'enceinte de traitement peut également comporter un tiroir ou un récipient interne amovible, permettant d'évacuer plus simplement et sans risques les déchets traités.

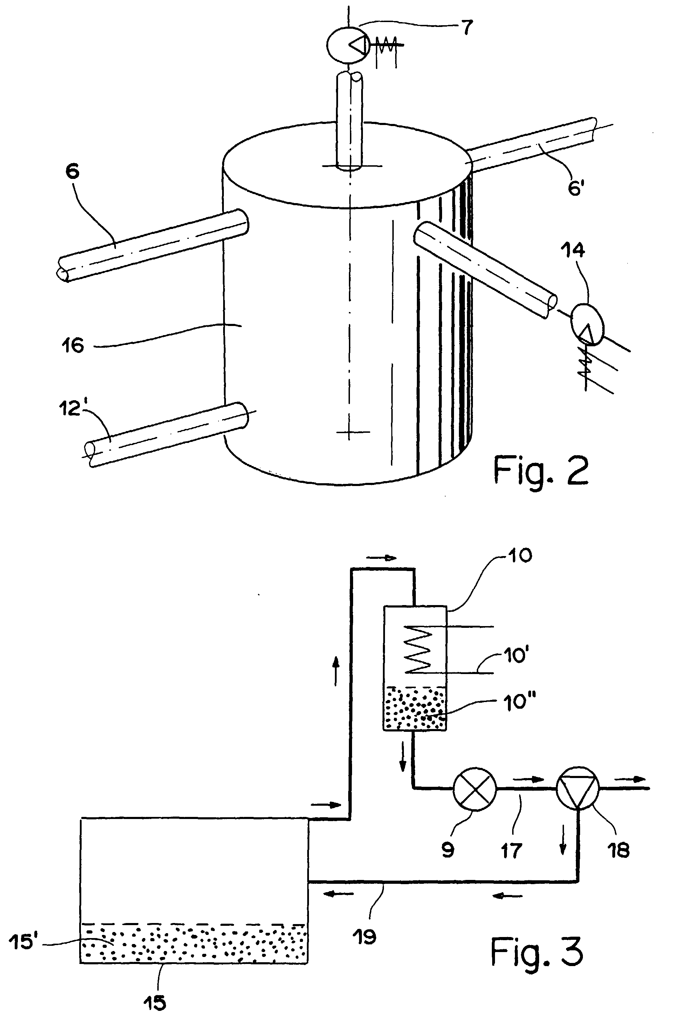

La figure 2 représente l'enceinte d'accumulation 16 selon l'invention. Elle est constituée d'un récipient hermétique 16 vertical, en acier inoxydable ou en tout autre matériau adapté. La forme générale verticale et les dimensions de l'enceinte sont prévues de façon à ce que l'air ozoné, en provenance directe du générateur 5', injecté par la tubulure 6 dans la partie supérieure de l'enceinte ait tendance à descendre, par simple gravité, et à s'accumuler dans la partie inférieure de l'enceinte. Un orifice de prélèvement relié à la tubulure d'injection 12' est prévu dans cette partie inférieure de l'enceinte d'accumulation 16.

Une tubulure reliée à l'électrovanne de mise à l'air libre 14 aboutit également dans la partie supérieure de l'enceinte d'accumulation. Lors des étapes d'injection, l'air externe parvient par cette tubulure et par l'électrovanne 14 dans l'enceinte d'accumulation 16, créant un effet de piston pneumatique aidant à chasser l'ozone vers l'enceinte de traitement 15. L'enceinte d'accumulation est encore munie dans sa partie supérieure d'une soupape de sûreté 7, évacuant l'excès d'air ozoné vers un destructeur d'ozone auxiliaire 8 (représenté sur la figure 1).

Dans une variante, un ensemble de tubulures et de vannes peut être prévu pour décharger l'excès d'air ozoné libéré par la soupape de sûreté 7 vers le destructeur d'ozone principal 10, évitant ainsi de devoir prévoir un destructeur d'ozone auxiliaire.

Dans une autre variante, les moyens de génération d'air ozoné 5 sont montés en circuit fermé avec l'enceinte d'accumulation 16, selon le principe décrit dans la demande de brevet déjà mentionnée WO94/06483. Le couplage de retour du gaz de l'enceinte 16 vers le générateur se fait alors également dans la partie supérieure de 16 par la tubulure 6'; la soupape de sûreté 7 et le destructeur d'ozone auxiliaire 8 ne sont dans ce cas pas nécessaires.

La figure 3 illustre une variante du circuit de purge de l'invention, monté cette fois en circuit fermé.

Dans des applications de stérilisation, la purge à l'air libre peut entraîner une recontamination secondaire des déchets, due aux micro-organismes contenus dans l'air frais ambiant. Pour pallier à cet inconvénient, dans cette variante, une vanne à trois voies 18 permet au cours de la purge de réinjecter dans l'enceinte de traitement 15 via une tubulure de réinjection 19 l'air aspiré par la pompe à vide 9 via le destructeur d'ozone 10, afin d'établir une destruction de l'ozone résiduel en boucle fermée. Cette disposition évite toute contamination extérieure, l'ozone résiduel étant progressivement recombiné en oxygène moléculaire.

La présente description a porté essentiellement sur un exemple de dispositif de décontamination applicable à l'élimination de déchets provenant de la pratique médicale à l'intérieur d'une enceinte de décontamination. L'invention est cependant adaptable sans modifications importantes au problème du traitement, de la désodorisation ou de la stérilisation de corps creux (tuyaux, cuves, récipients, emballages), notamment dans l'industrie agroalimentaire, pharmaceutique ou chimique. D'autres gaz de traitement que l'air ozoné peuvent en outre être utilisés selon l'application, et des additifs peuvent être ajoutés pour améliorer encore l'efficacité du traitement ou pour supprimer des odeurs..

## Revendications

1. Dispositif de traitement de matériaux par exposition à un gaz de traitement, notamment pour la décontamination, la désinfection et/ou ou la stérilisation, comportant:
des moyens de génération du gaz de traitement (5),
une enceinte d'accumulation du gaz produit (16),
une enceinte de traitement (15) dans laquelle les matériaux à traiter (15') peuvent être introduits,
un ensemble de tubulures reliant ces éléments les uns aux autres pour que le gaz de traitement accumulé dans l'enceinte d'accumulation (16) puisse parvenir dans l'enceinte de traitement (15),
caractérisé en ce que le dispositif comprend
des moyens (9) destinés à établir une dépression dans l'enceinte de traitement (15),
une vanne d'injection (12) sur la tubulure reliant l'enceinte d'accumulation (16) et l'enceinte de traitement (15),
une vanne (14) située en amont de l'enceinte d'accumulation (16) permettant à cette enceinte d'accumulation de communiquer temporairement avec l'atmosphère,
l'ouverture de la vanne d'injection (12) et la vanne de mise à l'air libre (14) étant asservies à la dépression établie dans l'enceinte de traitement (15) de manière à injecter brutalement le gaz de traitement accumulé et à rétablir la pression atmosphérique dans l'ensemble du système.

2. Dispositif selon la revendication 1, caractérisé en ce que le gaz de traitement utilisé est de l'air enrichi en ozone.

3. Dispositif selon la revendication 2, caractérisé en ce qu'il comporte en outre des moyens principaux de destruction d'ozone (10) pour détruire l'ozone résiduel quittant l'enceinte de traitement (15).

4. Dispositif selon la revendication 3, caractérisé en ce que lesdits moyens destinés à établir une dépression sont constitués par une pompe à vide (9) située en aval des moyens principaux de destruction d'ozone (10) et travaillant dans l'air.

5. Dispositif selon l'une des revendications 1 à 4, caractérisé en ce que les moyens de génération du gaz de traitement (5) sont placés en circuit ouvert avec l'enceinte d'accumulation (16).

6. Dispositif selon la revendication 1 à 5, caractérisé en ce que l'enceinte d'accumulation (16) est munie d'une soupape de sûreté (7) qui s'ouvre lorsque la pression dans l'enceinte d'accumulation dépasse un seuil prédéterminé afin d'évacuer la surpression vers un destructeur d'ozone (8; 10).

7. Dispositif selon l'une des revendications 1 à 6, caractérisé en ce que l'enceinte d'accumulation (16) est constituée d'un récipient étanche, le gaz de traitement provenant des moyens de génération (5) étant introduit dans la partie supérieure du récipient et évacué vers l'enceinte de traitement (15) par un orifice dans la partie inférieure du récipient.

8. Procédé de traitement de matériaux (15'), en vue notamment de la décontamination, de la désinfection et/ou de la stérilisation des matériaux, caractérisé en ce qu'il comporte plusieurs étapes distinctes d'injection d'un gaz de traitement dans une enceinte de traitement (15), et en ce qu'il est mis en oeuvre sur un dispositif selon l'une des revendications 1 à 6.

9. Procédé selon la revendication 8, caractérisé en ce qu'il fait appel à de l'air enrichi en ozone comme gaz de traitement.

10. Procédé selon la revendication 9, caractérisé en ce qu'il comporte avant chaque opération d'injection dans l'enceinte de traitement (15) d'air ozoné une étape de création d'une dépression dans l'enceinte de traitement.

11. Procédé selon la revendication 10, caractérisé en ce que l'injection d'air ozoné est effectuée partiellement par remise brutale en pression atmosphérique du dispositif.

12. Procédé selon l'une des revendications 8 à 11, caractérisé en ce qu'il comporte, en outre, entre chaque étape d'injection et de création d'une dépression, une étape d'agitation des matériaux à traiter (15') et/ou du milieu gazeux dans l'enceinte de traitement (15).

## Claims

1. Device for processing of materials by exposing to a processing gas, in particular for decontamination, disinfection and/or or *<sic.>* sterilisation, comprising:
means of generating the processing gas (5),
an enclosure for accumulation of the gas product (16),
a processing enclosure (15) in which the materials to be processed (15') can be introduced,
a set of connection pieces connecting these elements to one another so that the processing gas accumulated in the accumulation enclosure (16) can arrive in the processing enclosure (15),
characterised in that the device includes means (9) intended to establish a partial vacuum in the processing enclosure (15), an injection valve (12) on the connecting piece connecting the accumulation enclosure (16) and the processing enclosure (15), a valve (14) situated upstream from the accumulation enclosure (16) allowing this accumulation enclosure to communicate momentarily with the atmosphere, the aperture of the injection valve (12) and the vent valve (14) being subjected to the partial vacuum established in the processing enclosure (15) in such a way as to inject suddenly and forcefully the accumulated processing gas and to re-establish the atmospheric pressure in the whole system.

2. Device according to claim 1, characterised in that the processing gas used is ozone-enriched air.

3. Device according to claim 2, characterised in that it further comprises main ozone-destroying means (10) to destroy the residual ozone leaving the processing enclosure (15).

4. Device according to claim 3, characterised in that the said means intended to establish a partial vacuum are made up of a vacuum pump (9) situated downstream from the main ozone-destroying means (10) and working in the air.

5. Device according to one of the claims 1 to 4, characterised in that the means of generating the processing gas (5) are placed in open circuit with the accumulation enclosure (16).

6. Device according to the claim 1 to 5 *<sic.>,* characterised in that the accumulation enclosure (16) is equipped with a safety valve (7) which opens when the pressure in the accumulation enclosure surpasses a predetermined threshold in order to evacuate the excess pressure toward an ozone destroyer (8; 10).

7. Device according to one of the claims 1 to 6, characterised in that the accumulation enclosure (16) is made up of a tight receptacle, the processing gas coming from generating means (5) being introduced into the upper part of the receptacle and evacuated toward the processing enclosure (15) through an orifice in the lower part of the receptacle.

8. Process for processing of materials (15'), particularly in view of decontamination, disinfection and/or sterilisation of materials, characterised in that it comprises a plurality of distinct steps of injection of a processing gas into a processing enclosure (15), and in that it is implemented on a device according to one of the claims 1 to 6.

9. Process according to claim 8, characterised in that it uses ozone-enriched air as processing gas.

10. Process according to claim 9, characterised in that it includes before each of injection into the processing enclosure (15) of ozonised air a step of creating a partial vacuum in the processing enclosure.

11. Process according to claim 10, characterised in that the injection of ozonised air is carried out partially by suddenly and forcefully returning the device to atmospheric pressure.

12. Process according to one of the claims 8 to 11, characterised in that it further comprises between each step of injection and of creation of a partial vacuum, a step of stirring of the materials to be processed (15') and/or of the gaseous medium in the processing enclosure (15).

## Patentansprüche

1. Vorrichtung zur Behandlung von Materialien indem sie einem Behandlungsgas ausgesetzt werden, nämlich für die Dekontaminierung, die Desinfektion und/oder die Sterilisation, enthaltend:
Mittel zur Bildung des Behandlungsgases (5),
ein Akkumulationsgefäss für das erzeugte Gas (16),
ein Behandlungsgefäss (15) in welchem das zu behandelnde Material (15') eingeführt werden kann,
ein Rohrsystem, welches diese Elemente miteinander verbindet, damit das Behandlungsgas, das im Akkumulationsgefäss (16) akkumuliert wird, ins Behandlungsgefäss (15) gelangen kann,
dadurch gekennzeichnet, dass die Vorrichtung Mittel (9), die zur Erzeugung eines Unterdruckes im Behandlungsgefäss (15) bestimmt sind,
ein Einspritzventil (12) am Rohrsystem, welches das Akkumulationsgefäss (16) und das Behandlungsgefäss (15) verbindet,
ein Ventil (14), welches sich stromaufwärts vom Akkumulationsgefäss (16) befindet, welches es ermöglicht, dass dieses Akkumulationsgefäss temporär mit der Atmosphäre kommuniziert, enhält,
wobei, wenn die Öffnung des Injektionsventils (12) und das Ventil für die Verbindung mit der freien Luft (14) der Drucksenkung ausgesetzt werden, welche im Behandlungsgefäss (15) aufgebaut wird, das akkumulierte Gas schlagartig eingeleitet und der Atmosphärendruck in der Gesamtheit des Systems wiederum hergestellt wird.

2. Vorrichtung gemäss Anspruch 1, dadurch gekennzeichnet, dass das verwendete Behandlungsgas mit Ozon angereicherte Luft ist.

3. Vorrichtung gemäss Anspruch 2, dadurch gekennzeichnet, dass sie im Weiteren Ozonzerstörungsmittel (10) enthält, zur Zerstörung des restlichen Ozons, das aus dem Behandlungsgefäss (15) austritt.

4. Vorrichtung gemäss Anspruch 3, dadurch gekennzeichnet, dass die genannten Mittel zur Erzeugung eines Unterdruckes aus einer Vakuumpumpe (9) bestehen, die sich stromabwärts der Ozonzerstörungsmittel (10) befindet und in Luft arbeitet.

5. Vorrichtung gemäss einem der Ansprüche 1 bis 4, dadurch gekennzeichnet, dass die Mittel zur Bildung des Behandlungsgases (5) sich in einem offenen Kreis mit dem Akkumulationsgefäss (16) befinden.

6. Vorrichtung gemäss den Anspüchen 1 bis 5, dadurch gekennzeichnet, dass das Akkumulationsgefäss (16) mit einem Sicherheitsventil (7) ausgerüstet ist, welches sich öffnet, sobald der Druck im Akkumulationsgefäss eine vorbestimmte Schwelle überschreitet, damit der Überdruck in Richtung des Ozonzerstörers (8; 10) evakuiert wird.

7. Vorrichtung gemäss einem der Ansprüche 1 bis 6, dadurch gekennzeichnet, dass das Akkumulationsgefäss (16) aus einem dichten Behälter besteht, wobei das Behandlungsgas, das aus den Erzeugungsmitteln (5) kommt, in den oberen Teil des Behälters eingeführt wird und in Richtung des Behandlungsgefässes (15) durch eine Öffnung im unteren Teil des Gefässes evakuiert wird.

8. Verfahren zur Behandlung von Materialien (15'), insbesondere zur Dekontamination, Desinfektion/und oder Sterilisation von Materialien, dadurch gekennzeichnet, dass es mehrere verschiedene Schritte zur Injektion eines Behandlungsgases in ein Behandlungsgefäss (15) umfasst und dass es mit einer Vorrichtung gemäss einem der Ansprüche 1 bis 6 durchgeführt wird.

9. Verfahren nach Anspruch 8, dadurch gekennzeichnet, dass es als Behandlungsgas mit Ozon angereichterte Luft verwendet.

10. Verfahren nach Anspruch 9, dadurch gekennzeichnet, dass es vor jedem Schritt der Injektion von ozonhaltiger Luft in das Behandlungsgefäss (15) einen Schritt zur Bildung eines Unterdruckes im Behandlungsgefäss umfasst.

11. Verfahren nach Anspruch 10, dadurch gekennzeichnet, dass die Injektion von ozonhaltiger Luft teilweise dadurch erzielt wird, indem in der Vorrichtung zum Teil schlagartig wieder Atmosphäreneindruck hergestellt wird.

12. Verfahren gemäss einem der Ansprüche 8 bis 11, dadurch gekennzeichnet, dass es im Weiteren zwischen jedem Injektionsschritt und der Bildung eines Unterdrucks einen Mischschritt für die zu behandelnden Materialien (15') und/oder für das gasförmige Milieu im Behandlungsgefäss (15) umfasst.
